# EUROPEAN PATENT APPLICATION

(11) **EP 4 174 183 A1**
(43) Date of publication of application: **03.05.2023**
(21) Application number: 21894027.8
(22) Date of filing: 19.11.2021
(51) Int. Cl.: C12N 15/62, C07K 19/00, A61K 39/39, A61K 39/215, A61P 31/14, A61K 39/385

(54) **FUSION GENE, RECOMBINANT NOVEL CORONAVIRUS HIGH-EFFICIENCY IMMUNE DNA VACCINE, CONSTRUCTION METHOD THEREFOR AND USE THEREOF**

(30) Priority: 20.11.2020 CN 202011310160
(71) Applicant: Aurora Genevac Biotech Co., Ltd., Beijing 100085 (CN)
(72) Inventor: YU, Jiyun, Beijing 100085 (CN); WANG, Yu, Beijing 100085 (CN); SONG, Weiwei, Beijing 100085 (CN); YAN, Jinqi, Beijing 100085 (CN); XU, Qiang, Beijing 100085 (CN); XIA, Yu, Beijing 100085 (CN); LIU, Yachao, Beijing 100085 (CN); GAO, Kun, Beijing 100085 (CN); CHIOU, Chuang-Jiun, Beijing 100085 (CN)
(74) Representative: Abel & Imray LLP
(86) International application number: PCT/CN2021/131786
(87) International publication number: WO 2022/105880

(57) **Abstract**

A fusion gene, a recombinant novel coronavirus high-efficiency immune DNA vaccine, a construction method and use thereof are provided. The immune DNA vaccine ZD-nCor19 provided herein uses RBD protein, residues 301-538 in the S2 subunit and residues 138-369 in the N protein of the novel coronavirus as target antigens, and has specific immune synergism molecules introduced at suitable positions, and thus can simultaneously efficiently induce humoral immunity and cellular immunity, and can avoid safety problems associated with ADE that may be generated by the full-length S protein and the full-length N protein, thereby achieving dual effects of prevention and treatment. The vaccine can be used as a safe, efficient and stable vaccine variety against novel coronavirus infection.

## Description

### CROSS-REFERENCE TO RELATED APPLICATIONS

This application claims priority to Chinese Patent Application No. 202011310160.8, filed November 20, 2020, the content of which is incorporated by reference in its entirety.

### TECHNICAL FIELD

The present invention relates to the field of biomedical technology, in particular to a fusion gene, a recombinant novel coronavirus high-efficiency immune DNA vaccine (named ZD-nCor19), a construction method and use thereof.

### BACKGROUND OF THE INVENTION

Novel coronavirus (COVID-19) is a new strain of coronavirus that has never been found in human body before. The common signs of human infection with novel coronavirus include respiratory symptoms, fever, cough, shortness of breath and dyspnea. In more serious cases, the infection can lead to pneumonia, severe acute respiratory syndrome, renal failure, and even death.

So far, the cumulative number of people infected with novel coronavirus in the world has reached tens of millions, and the cumulative number of deaths has exceeded one million, posing a huge threat to the life, health and safety of all human beings. However, the COVID-19 pandemic is still spreading rapidly in many parts of the world. It has been confirmed that the virus is highly contagious, pathogenic and harmful. There are 7.8 billion people in the world who are at risk of infection, morbidity and death. It is internationally recognized that safe and effective vaccination is an important measure and effective means to protect human safety and health. Therefore, there is an urgent need to develop an effective and safe vaccine that can be used for both prevention and therapy against COVID-19.

At present, there are nearly 200 novel coronavirus vaccines in different stages of development worldwide, of which 10 novel coronavirus vaccines are undergoing phase III clinical trials (as of October 13, 2020), mainly including novel coronavirus inactivated vaccines, recombinant subunit vaccines, adenovirus vector vaccines, attenuated influenza virus vector vaccines, nucleic acid vaccines, etc.. Although phased results have been achieved in the research and development of novel coronavirus vaccines, there is still an urgent need for the development of novel coronavirus vaccine varieties that can be used for both prevention and treatment.

### SUMMARY

The present invention aims to provide a fusion gene and a recombinant novel coronavirus high-efficiency immune DNA vaccine and a construction method and use thereof.

In the first aspect of the present invention, a fusion gene comprising at least two of the following (1) to (4) is provided:
(1) a gene expressing RBD segment of novel coronavirus COVID-19;
(2) a gene expressing S2 subunit or partial fragment thereof of the novel coronavirus COVID-19;
(3) a gene expressing N protein or partial fragment thereof of the novel coronavirus COVID-19;
(4) a gene expressing fragment selected from the group consisting of CTB, TT, PADRE, Foldon, CPPCP, Furin2A, ERISS, IRES, and OX40L, or combination thereof.

In an embodiment, the fusion gene comprises at least three of the following (1) to (4):
(1) a gene expressing the RBD segment of the novel coronavirus COVID-19;
(2) a gene expressing residues 301-538 in the S2 subunit of the novel coronavirus COVID-19;
(3) a gene expressing residues 138-369 in the N protein of the novel coronavirus COVID-19;
(4) genes expressing the following amino acid fragments: CTB, TT, PADRE, Foldon, CPPCP, Furin2A, ERISS, IRES and OX40L.

In an embodiment, the gene expressing the RBD segment and the gene expressing the residues 301-538 in the S2 subunit are linked to form a fusion fragment; in a preferred embodiment, the nucleotide sequence of the fusion fragment comprises the sequence set forth in SEQ ID NO: 6; the nucleotide sequence of the gene expressing the residues 138-369 in the N protein comprises the sequence set forth in SEQ ID NO: 10.

In another embodiment, in the fusion gene:
the nucleotide sequence of the gene expressing the CTB amino acid fragment is set forth in SEQ ID NO: 2;
the nucleotide sequence of the gene expressing the TT amino acid fragment is set forth in SEQ ID NO: 3;
the nucleotide sequence of the gene expressing the PADRE amino acid fragment is set forth in SEQ ID NO: 4;
the gene expressing the Foldon amino acid fragment and the gene expressing the CPPCP amino acid fragment are linked to form a synthetic fragment, and the nucleotide sequence of the synthetic fragment is set forth in SEQ ID NO:7;
the nucleotide sequence of the gene expressing the Furin2A amino acid fragment is set forth in SEQ ID NO: 8;
the nucleotide sequence of the gene expressing the ERISS amino acid fragment is set forth in SEQ ID NO: 9;
the nucleotide sequence of the gene expressing the IRES amino acid fragment is set forth in SEQ ID NO: 11; and/or
the nucleotide sequence of the gene expressing the OX40L amino acid fragment is set forth in SEQ ID NO: 12.

In one embodiment, in the fusion gene: the gene expressing the RBD segment and the gene expressing the residues 301-538 in the S2 subunit are linked to form a fusion fragment, the upstream of the fusion fragment is sequentially linked with the genes expressing the amino acid fragments of CTB, TT and PADRE, and the downstream of the fusion fragment is sequentially linked with the genes expressing the amino acid fragments of Foldon, CPPCP and Furin2A; and/or the gene expressing the ERISS amino acid fragment is linked upstream of the gene expressing the residues 138-369 in the N protein, and the genes expressing the IRES and OX40L amino acid fragments are sequentially linked downstream of the gene expressing the residues 138-369 in the N protein.

In a preferred embodiment, the gene expressing the RBD segment and the gene expressing the residues 301-538 in the S2 subunit are linked by a gene expressing a (G4S)₂ linker, the upstream of the fusion fragment and the gene expressing the PADRE amino acid fragment are linked by a gene expressing a linker G6, wherein the nucleotide sequence of the gene expressing the linker G6is set forth in SEQ ID NO:5.

In further preferred embodiments, the nucleotide sequence of the fusion gene is set forth in SEQ ID NO: 13.

In the second aspect of the present invention, a fusion protein obtained by expressing the fusion gene described above is provided.

In the third aspect of the present invention, a recombinant novel coronavirus high-efficiency immune DNA vaccine, named ZD-nCor19, which comprises the above-mentioned fusion gene and vector, is provided.

In one embodiment, the vector is pZDVac vector.

The fourth aspect of the present invention also provides a method for constructing the above-mentioned recombinant novel coronavirus high-efficiency immune DNA vaccine, comprising the following steps of:
1) synthesizing the fusion gene described above;
2) inserting the fusion gene into a pZDVac vector to obtain the recombinant novel coronavirus high-efficiency immune DNA vaccine.

In the fifth aspect of the present invention, the use of the above-mentioned recombinant novel coronavirus high-efficiency immune DNA vaccine in the preparation of a medicine for preventing and/or treating novel coronavirus infection also belongs to the content of the present invention.

In the recombinant novel coronavirus high-efficiency immune DNA vaccine provided by the present invention, the RBD segment of S1 subunit, segment residues 301-538 in the S2 and segment residues 138-369 in the N protein was fused in frame and expressed as the target antigen. In addition, the fusion gene is further combined with other designs, for example, the upstream and downstream of the target antigen gene are respectively linked with gene expressing immune synergism molecule, and the Furin2A protease cleavage site is set between the genes expressing the target antigen. The above designs allow the recombinant novel coronavirus high-efficiency immune DNA vaccine (recombinant plasmid pZDVac-CRSNPO) constructed and obtained by the present invention can not only effectively avoid the ADE-related problems that may be generated by the full-length S protein and the full-length N protein, but also ensure that the fusion protein expressed by fusion gene can cover more epitopes of the novel coronavirus and achieve more comprehensive protection, compared with using only S1 protein or only RBD or only using S1 protein (RBD) and N protein as target antigens. In the design of the target antigen, the vaccine uses only RBD, residues 301-538 in the S2 subunit and residues 138-369 in the N protein, which have smaller fragments relative to the full-length S protein and N protein, not only beneficial to the construction of vaccines, but also make it easier to enter cells, and have high expression efficiency and high immune effect. In addition, the presence of the Furin2A protease cleavage site allows the extracellular secretion of two antigenic segments (RBD and residues 301-538 in the S2 subunit) derived from the S protein, thereby inducing a better humoral immune response, while the antigenic segment (residues 138-369 in the N protein) derived from the N protein can be expressed intracellularly to induce T cell immune response. Thus, the vaccine constructed and obtained by the present invention can efficiently induce humoral immune response and T cell immune response at the same time, and the two synergistically enhance the immune protection effect of the vaccine.

Compared with the prior art, the present invention has the following beneficial effects:
1) High safety: the vaccine of the present invention belongs to the RNA vaccine in the form of DNA, and is not integrated into the chromosome, so it will not cause malignant transformation of cells. Moreover, after the vaccine enters cells, RNA transcription may be carried out on a large scale, resulting in rapid apoptosis of cells, further avoiding the possibility of malignant transformation. In addition, the vaccine is a naked plasmid product, without additional liposomes, aluminum adjuvants, etc., and basically does not cause additional non-specific inflammatory responses. Therefore, in terms of safety, it is the most advantageous and most guaranteed among all types of vaccines.
2) High-efficiency immunity: the vaccine of the present invention belongs to a class of high-efficiency immune DNA vaccines ((ipDNA vaccine) ZD-nCor19) which combines the dual advantages of DNA vaccines and RNA vaccines. The verified results show that by using vaccination via intradermal injection + electrical pulse assisted introduction (two injections, 28 days apart, 1-4 weeks apart in emergencies), the vaccines may efficiently induce mucosal-like surface immune protective efficacy, and induce systemic high-efficiency cellular immune responses, which can not only form a strong superficial immune protection effect, but also have a strong deep protective effect. This kind of immune response belongs to the high-level immune response, which has the characteristics of `rapid immune decision mobilization', `group army annihilation campaign' and `active defense clearance in depth', providing efficient and lasting immune protection for the human body. Once attacked by the virus, systemic immune mobilization can be carried out quickly and efficiently, including the immune response at the superficial mucosal level, as well as systemic innate immunity, humoral immunity and cellular immunity, to quickly kill and clear the novel coronavirus invading the body, prevent the virus from causing disease and increasing serious disease condition, protect life, avoid various sequelae, and complete the efficient killing and clearance of viruses while the body does not produce symptoms or only produces mild symptoms, thus quickly restore health.

In addition, the high-efficiency immunity of the vaccine is also reflected in the ability to enhance the systemic immunity in human bodies. For the elderly with weak immunity and people with serious underlying diseases, the vaccine is an excellent life-protecting vaccine, helping to protect the life and health of the elderly and immune-vulnerable groups.
3) Long-acting immunity and anti-variants: in addition to the high-efficiency immune protection feature of the vaccine of the present invention, the antigen-specific cellular immunity induced by the vaccine will retain the ability of immune memory for years, so the vaccine is also a vaccine that can provide long-acting immune protection. Moreover, in the vaccine design, a partial fragment of the internal N protein of the virus is used as the target antigen, which is extremely stable and rarely produces confirmed mutation, thus the vaccine of the present invention is also a vaccine that can effectively resist virus mutation. Even after some vaccines lose their immune protection efficacy due to the significant variation of virus surface antigens, the vaccine of the present invention can still provide high-efficiency immune protection.
4) Used for both prevention and therapy: the vaccine of the present invention can not only be used as a preventive vaccine, but also can be used for emergency immunotherapy for people who test positive for the virus. The vaccine can induce a high-efficiency cellular immune response quickly, clean the virus in the body in a timely and efficient manner, prevent the condition from getting worse, help save lives, and reduce the fatality rate of the virus. Therefore, the vaccine of the present invention will be a vaccine that can truly protect and save lives.
5) Large manufacturing capacity and low cost: the vaccine of the present invention can use the *E. coli* prokaryotic system for biological fermentation production, so the existing biological fermentation industrial system can be used to establish a new industrial system, so the production of the vaccine can form a huge manufacturing capacity, and the cost is relatively lower. Therefore, it is expected to meet domestic and international demand for this vaccine variety.
6) Highly stability: DNA vaccines generally have strong stability and can be stored and transported at room temperature, while RNA vaccines, protein subunit vaccines and viral vector vaccines, as well as inactivated vaccines require cold chain transportation, which is a particular advantage of DNA vaccines over other types of vaccine varieties. The vaccine provided is a DNA vaccine, thus it has a very great advantage in stability, and can become an excellent choice among the national novel coronavirus vaccine foreign-aid varieties.
7) Broad market: At present, there is no novel coronavirus vaccine on the market against Novel coronavirus, therefore, the effect of the current clinical or under development vaccines cannot be judged. It is necessary to use a variety of vaccines under development to make a "combined boxing" to form a vaccine complementation. The vaccine herein can not only be used alone to provide high-efficiency protection against Novel coronavirus, for other types of vaccines, it can also be used for booster immunization, to provide enhanced immune protection in conjunction with other types of vaccines. Furthermore, the vaccine provided herein can even help some vaccines that may have potential ADE effect (antibody-dependent enhancement) overcome the possible ADE effect, and avoid the serious problem of ADE effect aggravating the disease.

In conclusion, the vaccine (ipDNA vaccine) ZD-nCor19 provided herein is expected to have the characteristics and advantages of safety, high efficiency, long-acting, anti-mutation, highly stability and huge manufacturing capacity. It is a DNA vaccine with efficient immune effect, and can be used for routine prevention in population, specific immune protection of immune vulnerable groups such as the elderly, immune enhancement of specific groups, emergency vaccination after virus exposure, coordinated immune enhancement of different types of vaccines, and as a national foreign aid vaccine. As an excellent vaccine for both prevention and therapy, the vaccine is expected to be further developed and utilized.

### DISCRIPTION OF DRAWINGS

Figure1 is a schematic diagram showing the connection structure of target antigens and immune synergism molecules in a recombinant novel coronavirus high-efficiency immune DNA vaccine provided by an example of the present invention.
Figure 2 is a gel electrophoresis photograph of double digested pZDVac-CCCPO plasmid.
Figure 3 is a gel electrophoresis photograph of double digested pUC57-CRSNP plasmid.
Figure 4 is a gel electrophoresis photograph showing the recovery and identification of the target gene fragment and vector backbone of the recombinant plasmid pZDVac-CRSNPO.
Figure5 is a construction schematic diagram of recombinant plasmid pZDVac-CRSNPO.
Figure 6 is a gel electrophoresis photograph showing identification of positive clones by colony PCR.
Figure 7 is a gel electrophoresis photograph showing digestion identification of the plasmid extracted from positive clones on a small amount.
Figure 8 is a bar graph of the secretion frequency of cytokines IFN-γ and IL-4 in mouse spleen cells with N protein as a stimulator after immunizing mice with recombinant plasmid pZDVac-CRSNPO.
Figure 9 is a bar graph of the secretion frequency of cytokines IFN-γ and IL-4 in mouse spleen cells with RBD protein as a stimulator after immunizing mice with recombinant plasmid pZDVac-CRSNPO.
Figure 10 is a scatter plot of the type bias analysis of T cell responses induced by recombinant plasmid pZDVac-CRSNPO in a mouse model.
Figure 11 is a scatter plot of the secretion of cytokine IFN-γ in human PBMCs with N protein and RBD protein as stimulators before and after immunization of human subjects with recombinant plasmid pZDVac-CRSNPO.
Figure 12 is a scatter plot of the secretion of cytokine IFN-γ in human PBMCs with N protein and RBD protein as stimulators before and after immunization of human subjects with recombinant plasmid pZDVac-CRSNPO at different doses.
Figure 13 is a scatter diagram of the detection results of antibodies induced by using the recombinant plasmid pZDVac-CRSNPO to immunize mice under different immunization routes and different electro-introduction conditions.
Figure 14 is a bar chart of the Elispot assay results of N antigen, RBD antigen and S2 antigen induced after immunizing mice with recombinant plasmid pZDVac-CRSNPO under different immunization routes and different electro-introduction conditions.

### DETAILED DESCRIPTION OF THE INVENTION

The present invention aims to provide a recombinant novel coronavirus high-efficiency immune DNA vaccine (ipDNA vaccine) ZD-nCor19 having both preventive and therapeutic functions, as well as construction method and use thereof, which are mainly realized based on the following technical means.

An alphavirus replicase-based DNA (replicon) vector pZDVac is used in the construction of the recombinant novel coronavirus high-efficiency immune DNA vaccine. The pZDVac vector is obtained from the existing pSFV1 plasmid through the following modifications: replacing the original SP6 promoter with the human cytomegalovirus CMV promoter, adding a polyadenylic acid tail SV40 poly(A), and replacing the resistance gene of the pSFV1 plasmid with a kanamycin resistance gene with clinically better safety, and the replicon after the above successful modifications is finally expressed in eukaryotes, and the resulted vector is named pZDVac vector(namely pSFVK1 disclosed in the patent document CN105343874A). By using the vector, tedious processes of preparing RNA in vitro is avoided. The vector can be directly transfected into human and animal cells in the form of DNA plasmids, achieving high-level expression of foreign proteins. The vector inherits a function that alphavirus vectors can induce the production of type I IFN to provide adjuvant effect for the expressed antigen, and which has the effect of enhancing immunogenicity.

The following factors in antigen design are fully considered, and optimized design schemes are proposed:
(1) at present, there is still little basic research on the novel coronavirus, and no novel coronavirus vaccine on the market against Novel coronavirus, therefore, there is no exact standard for the selection of the target antigen for the novel coronavirus vaccine, that is, there is no basis to determine which segment of the novel coronavirus antigen is more effective as the vaccine target antigen. Therefore, researchers generally select novel coronavirus antigens based on the development of SARS virus vaccine, and in target antigens selection, researchers generally use the full-length spike glycoprotein (Spike Protein, S protein, which consists of two subunits, S 1 and S2) or truncated S protein (S 1 subunit and its receptor binding region RBD) form, and the highly conserved nucleocapsid protein N (SARS-CoV-2 nucleocapsid) in the novel coronavirus. It is commonly believed that it is safer to choose the full-length S protein, which contains most of the antigenic epitopes and has a wider coverage; RBD is chosen because it contains a critical neutralizing domain (CND), which can induce potent neutralizing antibody response and possible cross-protection against mutant strains; N protein is chosen because it is a highly immunogenic and conserved phosphoprotein among coronaviruses, which can not only induce specific antibodies, but also specific cytotoxic cell activity, and it is highly conservative among various coronaviruses, so it is relatively less likely to mutate, which can avoid the risk of vaccine failure due to virus mutation (S protein is an antigen that is more likely to mutate in the virus, and if the virus mutates, it is likely to affect the immune efficacy of the vaccine.). Among them, according to the structure of the novel coronavirus and the mechanism of infecting patients, it is generally believed that the S 1 subunit and N protein of the novel coronavirus are the main viral antigens that trigger strong immune responses, and are the best viral antigens for vaccine development.
(2) The inventors have considered that the full-length S protein and full-length N protein of the novel coronavirus may cause ADE-associated problems. Therefore, the inventors optimized the design and finally determined that the RBD of the S 1 subunit of the S protein, residues 301-538 in the S2 subunit and residues 138-369 in the N protein was chosen for target antigen design. Among them, the RBD of the S 1 subunit contains the key neutralizing region, which is the key to vaccine development; residues 301-538 in the S2 subunit contains most of the T cell epitopes, and residues 138-369 in the N protein also contains multiple T cell antigen epitopes. Such a target antigen design can not only effectively avoid the ADE-related problems that may be caused by the full-length S protein and the full-length N protein, but also ensure that the designed fusion protein covers more antigen epitopes, achieving more comprehensive protection, relative to those using S 1 protein only or those using RBD only or those using the fusion protein of S 1 protein (or RBD) and N protein. At the same time, in the design of the target antigen of the present invention, the RBD, residues 301-538 in the S2 subunit and residues 138-369 in the N protein, which have smaller fragments relative to the full-length S protein and N protein, are used. This design of the target antigen is not only conducive to the construction of vaccines, but also makes it easier to enter into cells, and has high expression efficiency and high immune effect. In addition, in the target antigen design, two antigen segments (RBD and residues 301-538 in the S2 subunit) derived from the S protein are designed for secretory expression, which could induce better humoral immune responses; an antigenic segment (residues 138-369 in the N protein) derived from N protein is designed to be expressed intracellularly, which can further enhance the induced T cell immune response, thus the vaccine constructed based on this design can efficiently induce humoral immune response and T cell immune response simultaneously, and the two synergistically enhance the immune protection effect of the vaccine.
(3) The inventors have also considered that the immunogenicity of the DNA vaccine or viral vector vaccine using antigens alone might be insufficient. Therefore, in order to further improve the immunogenicity of the recombinant novel coronavirus DNA vaccine obtained by construction, and thereby improve its immune protection efficacy, immune synergism molecules suitable for the target antigen in the vaccine constructed by the present invention were also screened and determined , including:
   (i) cholera toxin B subunit (CTB): it can initiate innate immunity, activate DC, enhance immune response, help transfer antigens into cells, induce TH1 and TH2 immune pathways, and act as mucosal immune adjuvant;
   (ii) tetanus toxin helper T cell epitopes (TT) and pan-DR helper T cell epitopes (PARDE): they can enhance CD4+ T cell responses;
   (iii) Foldon-CPPCP: Foldon is a domain that enables non-covalent oligomerization of target proteins. It is derived from the C-terminus of T4 bacteriophage fibrin and consists of 27 amino acids. The oligomerization structure formed by non-covalent force has high stability. The structure will depolymerize when the temperature is higher than 75 °C or in more than 2% SDS at room temperature, and after depolymerization, it can still re-form trimers in a suitable environment. CPPCP (cysteine-proline-proline-cysteine-proline) is similar to antibody hinge region, suitable for bispecific antibodies and fusion proteins, and is easy to granulate the trimeric proteins. This type of immune potentiating molecule can make the antigen expressed by the vaccine form multimers and granules, which can further enhance its immunogenicity;
   (iv) ERISS sequence: it is the adenovirus E3 leader sequence, which is an endoplasmic reticulum insertion signal sequence, namely ER insertion signal sequence (ERISS), which can bind to the endoplasmic reticulum signal recognition particle (SRP). Through the characteristic that SRP can bind to the SRP receptor on the surface of the ER membrane, the fusion target antigen can be effectively transported into the ER, and the processing of the antigen by APC and the presentation of MHC class I molecules are enhanced, thereby enhancing the induction of effective protective CTL production;
   (v) OX40L: it can provide costimulatory signals for T cell activation.

A series of fusion genes can be obtained by integrating part or all of the genes expressing these immunopotentiating molecules into any one of the genes expressing the RBD, residues 301-538 in the S2 subunit and residues 138-369 in the N protein or a combination thereof. These fusion genes are inserted into the determined pZDVac vector to construct and obtain the recombinant novel coronavirus DNA vaccine of the present invention.

Fusion genes include various combinations (the following description is based on the protein structure after fusion gene expression), such as:
Combination 1: RBD protein and residues 301-538 in the S2 subunit are linked together through (G4S)₂ linker, and then cholera toxin B subunit, tetanus toxin helper T cell epitopes (TT) and pan-DR helper T cell epitopes(wherein the pan-DR helper T cell epitopes and RBD proteins are linked by G6 flexible linker) are sequentially linked to the amino terminus (N terminus) of RBD protein. Surprisingly, relative to other linking sequences (for example, tetanus toxin helper T-cell epitope, pan-DR helper T-cell epitope, and cholera toxin B subunit, or tetanus toxin helper T-cell epitope, cholera toxin B subunit and pan-DR helper T cell epitope, etc.), the linking sequence of the three immunepotentiating molecules mentioned above is more conducive to display the target antigen and enhance the immune response induced by the target antigen.
Combination 2: Foldon-CPPCP is linked to the carboxy-terminus (C-terminus) of residues 301-538 in the S2 subunit. The function of Foldon is to easily trimerize the antigens, and CPPCP is to further granulate the trimeric antigens. The combination of Foldon and CPPCP makes it easier to multimerize and granulate the target antigen after it is expressed. Compared with soluble antigens, it is easier to be phagocytosed by APC, and can activate Th cells, CTL cells and B cells at the same time, which can stimulate both cellular immunity and humoral immunity, so it can generate a comprehensive immune response and greatly enhance the immune protection effect of the vaccine. In this combination, the inventors unexpectedly find that in relative to other linking sequences (e.g. CPPCP-Foldon) and other positions, combining the two adjuvant molecules, Foldon and CPPCP, and placing them after the fusion fragment of RBD protein and residues 301-538 in the S2 subunit in the linking sequence of Foldon-CPPCP are more conducive to the display of Foldon and CPPCP after expression. If the adjuvant molecules Foldon-CPPCP are placed in other positions of the fusion fragment, it is likely to cause the two adjuvant molecules to be easily encapsulated in the middle of the antigen protein due to their small molecular weight after expression. Thus, the spatial structure of the two adjuvant molecules will not be displayed, and they will not play the role of polymerizing and granulating the target antigen after expression. Combination of Combination 2 and Combination 1 can produce a stacking effect or a synergistic effect.
Combination 3: at the carboxy terminus of CPPCP based on Combination 2 or at the carboxy terminus of residues 301-538 in the S2 subunit based on Combination 1, Furin2A, ERISS and residues 138-369 in the N proteinare sequentially linked. Among them, ERISS can enhance the processing of antigens by APC and the presentation by MHC class I molecules, thereby enhancing the T cell immune response of the N protein segment expressed in cells.Furin2A is a protease cleavage site, which can make residues 138-369 in the N protein independently expressed intracellularly to induce a T cell immune response.
Combination 4: based on combination 3, an internal ribosome entry site (IRES) sequence is linked to the C-terminus of residues 138-369 in the N protein, and downstream of the IRES sequence, an OX40L molecule is further linked, which can deliver a second signal for T cell activation, further stimulate the immune response, and induce the immune response to shift to Th1 type high-level immune response.

In addition, a Kozak sequence and a signal peptide sequence (which can also be added to the backbone vector in advance) can be sequentially introduced upstream of the fusion genes in each combination above-mentioned, and the signal peptide can be selected from signal peptides suitable for eukaryotic expression.

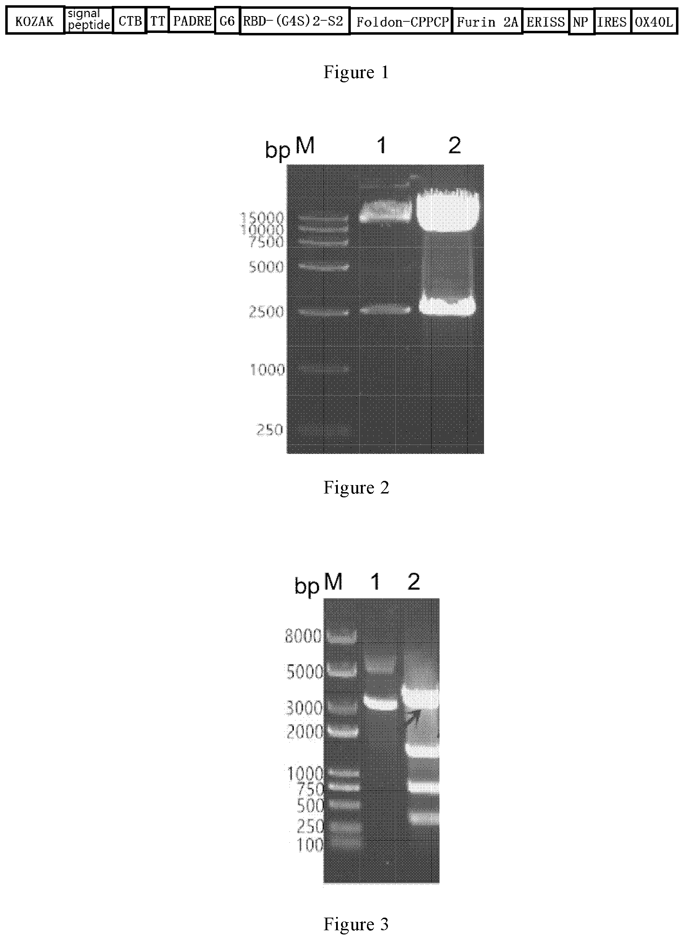

Figure 1 shows an example of the connection structure of the target antigens and immune potentiating molecules in the recombinant novel coronavirus high-efficiency immune DNA vaccine, including the fusion gene integrating the above Combination 1 - Combination 4 and the Kozak sequence and signal peptide sequence upstream of the fusion gene.

The design of the structure of the recombinant novel coronavirus DNA vaccine provided by a preferred embodiment of the present invention can generate synergy among the target antigens, among the immunopotentiating molecules, and between the target antigens and the immunopotentiating molecules, so as to improve the immune protection effect of the DNA vaccine. At the same time, the DNA vaccine is designed to express the segments (the RBD and residues 301-538 in the S2 subunit) of S protein and the segment (residues 138-369) of N protein, respectively, in which the S protein segment is the extracellular expression form, and the N protein segment is the intracellular expression form, therefore, they can induce good humoral immune response and T cell immune response at the same time, and can further improve the immune protection effect of the DNA vaccine.

The content of the present invention will be described in detail below with reference to specific examples and accompanying drawings.

In the following, certain exemplary examples are briefly described. As those skilled in the art would realize, the described examples may be modified in various different ways, all without departing from the spirit or scope of the present invention. Accordingly, the drawings and descriptions are to be regarded as illustrative in nature and non-limiting.

The methods used in the following examples are conventional methods unless otherwise specified, and the specific steps can be found in: "Molecular Cloning: A Laboratory Manual" Sambrook, J., Russell, David W., Molecular Cloning: A Laboratory Manual, 3rd edition, 2001, NY, Cold Spring Harbor.

The ways of obtaining various biological materials described in the examples are only to provide a way to obtain experimentally to achieve the purpose of specific disclosure, and should not be a limitation on the source of the biological materials of the present invention. In fact, the sources of the used biological materials are extensive, and any biological materials that can be obtained without violating laws and ethics can be replaced and used according to the teachings in the examples.

All primers and sequences mentioned in the present invention were synthesized using prior art.

### Example 1: Construction of recombinant novel coronavirus high-efficiency immune DNA vaccine ZD-nCor19

In this example, the pZDVac vector was used as the carrier of the DNA vaccine, the RBD, segment (residues 301-538) in the S2 subunit and segment (residues 138-369) in the N protein of the novel coronavirus were used as the target antigens, and cholera toxin B, tetanus toxin helper T cell epitopes, pan-DR helper T cell epitopes, Foldon-CPPCP, Furin2A, ERISS, IRES and OX40L (the respective corresponding nucleotide sequences are shown in Table 1 below, wherein the RBD and segment (residues 301-538) in the S2 subunit was linked by (G4S)₂ (RBD-(G4S)₂-S2), Linker G6 in Table 1 is indicated as the linker sequence between RBD and PARDE, the nucleotide sequence of segment (residues 138-369) in the N protein is given as NP in Table 1) were also used as immune synergism molecules to construct a recombinant novel coronavirus high-efficiency immune DNA vaccine. The construction method specifically includes the following steps.

### 1.1. Preparation and identification of expression vector backbone

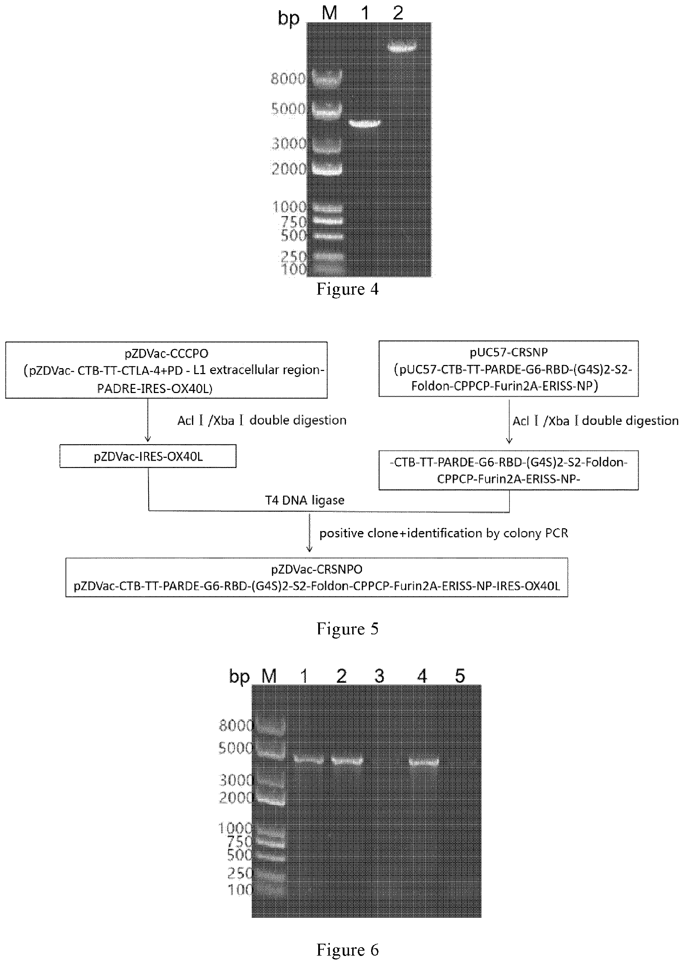

The pZDVac-CCCPO plasmid (which was obtained by cloning the synthetic gene CTB-TT-CTLA-4+PD-L1 extracellular region-PADRE-IRES-OX40L into a pZDVac vector, and its structure was pZDVac-CTB-TT-CTLA-4+PD-L1 extracellular region-PADRE-IRES-OX40L, the corresponding nucleotide sequence is set forth in SEQ ID NO: 1) was digested by *AclI* and *Xbal* restriction enzymes, and the photo of 0.45% gel electrophoresis was shown in Figure 2,wherein Lanes 1 and 2 were the results of the pZDVac-CCCPO plasmid after double digestion with *AclI* and *Xbal.* The recovered large fragment of about 12 kb was pZDVac-IRES-OX40L. The result of re-gel electrophoresis identification of the recovered fragment was shown in Lane 2in Figure 4, and it could be used as an expression vector backbone for constructing a novel coronavirus high-efficiency immune DNA vaccine.

### 1.2. Synthesis and identification of gene fragments

As shown in Figure 1, gene synthesis was carried out according to the sequence of gene connection of CTB-TT-PARDE-G6-RBD-(G4S)₂-S2-Foldon-CPPCP-Furin2A-ERISS-NP (named as first target gene, CRSNP for short), and the restriction sites *XbaI, NruI, PmeI* were introduced upstream of the first target gene, and a single restriction site *AclI* was introduced downstream of the first target gene, thus a recombinant plasmid(named pUC57-CRSNP recombinant plasmid) carrying the target gene fragment CRSNP was obtained. The steps were performed by Beijing Biomed Gene Co., Ltd..

The pUC57-CRSNP recombinant plasmid was digested by *AclI* and *Xbal* restriction enzymes (purchased from New England Biolabs (NEB) Inc.), and the photo of 0.45% agarose gel electrophoresis (Regular Agarose G-10, from Biowest company) was shown in Figure 3, wherein Lane 1 showed the electrophoresis results of the pUC57-CRSNP recombinant plasmid, and Lane 2 showed the electrophoresis results of the pUC57-CRSNP recombinant plasmid after double digestion with *AclI* and *XbaI.* The target gene fragment CRSNP (as shown by the arrow in Figure 3, named as first target gene fragment)was obtained by recovering (Agarose Gel DNA Recovery Kit (Spin-Columns) from TIANGEN) the target fragment of 3.6kb. The result of re-gel electrophoresis identification of the recovered first target gene fragment was shown in Lane 1in Figure 4.In this example, the inventors also synthesized two other target gene fragments: RBD-(G4S)₂-S2-NP (named as second target gene fragment, which differs from the first target gene fragment only in that it was not linked to immune synergism molecules) and Foldon-CPPCP-TT-PARDE-G6-RBD-(G4S)₂-S2-CTB-Furin2A-ERISS-NP (named third target gene fragment, which differs from the first target gene fragment in the positions of the immune adjuvant molecules, Foldon-CPPCP and CTB).

**Table 1: Nucleotide sequence information of each structure in the recombinant novel coronavirus high-efficiency immune DNA vaccine**

| Name | Nucleotide sequences |
|---|---|
| cholera toxinB (CTB) | ATGATCAAGCTGAAGTTCGGGGTGTTCTTCACCGTGCTGCT GAGCAGCGCCTACGCCCACGGGACCCCTCAGAACATCACC GACCTGTGCGCCGAGTACCACAACACCCAGATCTACACCCT GAACGACAAGATTTTCAGCTACACCGAAAGCCTGGCAGGA AAAAGAGAGATGGCCATCATCACCTTCAAAAACGGCGCCA TCTTCCAGGTGGAGGTGCCCGGCAGCCAGCACATCGATAG CCAGAAGAAGGCCATCGAAAGAATGAAGGACACACTGAG AATCGCCTACCTGACCGAGGCCAAGGTGGAAAAGCTGTGC GTGTGGAATAATAAGACCCCCCACGCCATCGCCGCCATCA GCATGGCCAAC（SEQ ID NO:2） |
| tetanus toxin helper T cell epitopes(TT) | CAGTACATCAAGGCAAACAGCAAATTCATCGGAATCACCG AA（SEQ ID NO:3） |
| pan-DR helper T cell epitopes (PARDE) | GCCAAATTTGTGGCCGCCTGGACCCTGAAGGCAGCCGCT （SEQ ID NO:4） |
| Linker G6 | GGCGGCGGCGGCGGCGGC (SEQ ID NO:5) |
| S antigen RBD-(G4S)₂-S2 | CGCGTGCAGCCCACCGAGAGCATCGTGCGCTTCCCCAACAT CACCAACCTGTGCCCCTTCGGCGAGGTGTTCAACGCCACCC GCTTCGCCAGCGTGTACGCCTGGAACCGCAAGCGCATCAG CAACTGCGTGGCCGACTACAGCGTGCTGTACAACAGCGCC AGCTTCAGCACCTTCAAGTGCTACGGCGTGAGCCCCACCAA GCTGAACGACCTGTGCTTCACCAACGTGTACGCCGACAGCT TCGTGATCCGCGGCGACGAGGTGCGCCAGATCGCCCCCGG CCAGACCGGCAAGATCGCCGACTACAACTACAAGCTGCCC GACGACTTCACCGGCTGCGTGATCGCCTGGAACAGCAACA ACCTGGACAGCAAGGTGGGCGGCAACTACAACTACCTGTA CCGCCTGTTCCGCAAGAGCAACCTGAAGCCCTTCGAGCGC GACATCAGCACCGAGATCTACCAGGCCGGCAGCACCCCCT GCAACGGCGTGGAGGGCTTCAACTGCTACTTCCCCCTGCAG AGCTACGGCTTCCAGCCCACCAACGGCGTGGGCTACCAGC CCTACCGCGTGGTGGTGCTGAGCTTCGAGCTGCTGCACGCC CCCGCCACCGTGTGCGGCCCCAAGAAGAGCACCAACCTGG TGAAGAACAAGTGCGTGAACTTCGGCGGCGGCGGCAGCGG CGGCGGCGGCAGCGTGCTGAACGACATCCTGAGCCGCCTG GACAAGGTGGAGGCCGAGGTGCAGATCGACCGCCTGATCA CCGGCCGCCTGCAGAGCCTGCAGACCTACGTGACCCAGCA GCTGATCCGCGCCGCCGAGATCCGCGCCAGCGCCAACCTG GCCGCCACCAAGATGAGCGAGTGCGTGCTGGGCCAGAGCA AGCGCGTGGACTTCTGCGGCAAGGGCTACCACCTGATGAG CTTCCCCCAGAGCGCCCCCCACGGCGTGGTGTTCCTGCACG TGACCTACGTGCCCGCCCAGGAGAAGAACTTCACCACCGC CCCCGCCATCTGCCACGACGGCAAGGCCCACTTCCCCCGCG AGGGCGTGTTCGTGAGCAACGGCACCCACTGGTTCGTGAC CCAGCGCAACTTCTACGAGCCCCAGATCATCACCACCGAC AACACCTTCGTGAGCGGCAACTGCGACGTGGTGATCGGCA TCGTGAACAACACCGTGTACGACCCCCTGCAGCCCGAGCT GGACAGCTTCAAGGAGGAGCTGGACAAGTACTTCAAGAAC CACACCAGCCCCGACGTGGACCTGGGCGACATCAGCGGCA TCAACGCCAGCGTGGTGAACATCCAGAAGGAGATCGACCG CCTGAACGAGGTGGCCAAGAACCTGAACGAGAGCCTGATC GACCTGCAGGAGCTGGGCAAGTACGAGCAGTACATCAAGT GGCCC（SEQ ID NO:6） |
| Foldon-CPPCP | GGCTACATCCCCGAGGCCCCCCGCGACGGCCAGGCCTACG TGCGCAAGGACGGCGAGTGGGTGCTGCTGAGCACCTTCCT |
| | GTGCCCCCCCTGCCCC（SEQ ID NO:7） |
| Furin 2A | CGCGCCAAGCGCGCCCCCGTGAAGCAGACCCTGAACTTCG ACCTGCTGAAGCTGGCCGGCGACGTGGAGAGCAACCCCGG CCCC（SEQ ID NO:8） |
| ERISS | ATGCGCTACATGATCCTGGGCCTGCTGGCCCTGGCCGCCGT GTGCAGCGCC（SEQ ID NO:9） |
| NP | GCCCTGAACACCCCCAAGGACCACATCGGCACCCGCAACC CCGCCAACAACGCCGCCATCGTGCTGCAGCTGCCCCAGGG CACCACCCTGCCCAAGGGCTTCTACGCCGAGGGCAGCCGC GGCGGCAGCCAGGCCAGCAGCCGCAGCAGCAGCCGCAGCC GCAACAGCAGCCGCAACAGCACCCCCGGCAGCAGCCGCGG CACCAGCCCCGCCCGCATGGCCGGCAACGGCGGCGACGCC GCCCTGGCCCTGCTGCTGCTGGACCGCCTGAACCAGCTGGA GAGCAAGATGAGCGGCAAGGGCCAGCAGCAGCAGGGCCA GACCGTGACCAAGAAGAGCGCCGCCGAGGCCAGCAAGAA GCCCCGCCAGAAGCGCACCGCCACCAAGGCCTACAACGTG ACCCAGGCCTTCGGCCGCCGCGGCCCCGAGCAGACCCAGG GCAACTTCGGCGACCAGGAGCTGATCCGCCAGGGCACCGA CTACAAGCACTGGCCCCAGATCGCCCAGTTCGCCCCCAGCG CCAGCGCCTTCTTCGGCATGAGCCGCATCGGCATGGAGGTG ACCCCCAGCGGCACCTGGCTGACCTACACCGGCGCCATCA AGCTGGACGACAAGGACCCCAACTTCAAGGACCAGGTGAT CCTGCTGAACAAGCACATCGACGCCTACAAGACCTTCCCCC CCACCGAGCCCAAGTGA（SEQ ID NO:10） |
| IRES | GCCCCTCTCCCTCCCCCCCCCCTAACGTTACTGGCCGAAGC CGCTTGGAATAAGGCCGGTGTGCGTTTGTCTATATGTTATT TTCCACCATATTGCCGTCTTTTGGCAATGTGAGGGCCCGGA AACCTGGCCCTGTCTTCTTGACGAGCATTCCTAGGGGTCTT TCCCCTCTCGCCAAAGGAATGCAAGGTCTGTTGAATGTCGT GAAGGAAGCAGTTCCTCTGGAAGCTTCTTGAAGACAAACA ACGTCTGTAGCGACCCTTTGCAGGCAGCGGAACCCCCCACC TGGCGACAGGTGCCTCTGCGGCCAAAAGCCACGTGTATAA GATACACCTGCAAAGGCGGCACAACCCCAGTGCCACGTTG TGAGTTGGATAGTTGTGGAAAGAGTCAAATGGCTCTCCTCA AGCGTATTCAACAAGGGGCTGAAGGATGCCCAGAAGGTAC CCCATTGTATGGGATCTGATCTGGGGCCTCGGTGCACATGC TTTACATGTGTTTAGTCGAGGTTAAAAAAACGTCTAGGCCC CCCGAACCACGGGGACGTGGTTTTCCTTTGAAAAACACGA （SEQ ID NO:11） |
| OX40L | ATGGAGCGGGTGCAGCCCCTGGAGGAGAACGTGGGCAACG CCGCCAGACCCAGGTTCGAGAGAAACAAGCTGCTGCTGGT GGCCAGCGTGATCCAGGGACTGGGCCTGCTGCTGTGCTTCA CCTATATTTGCCTGCACTTTAGTGCCCTGCAGGTGAGTCAT AGATACCCCCGCATTCAGTCCATCAAAGTGCAGTTCACCGA GTATAAGAAGGAGAAGGGATTCATTCTGACCTCCCAGAAG GAGGACGAGATCATGAAGGTGCAGAACAATAGCGTGATCA TCAACTGTGACGGCTTTTACCTGATTTCACTGAAAGGATAC TTCAGCCAGGAGGTGAATATCAGCCTGCACTACCAGAAAG ACGAGGAGCCCCTGTTCCAGCTGAAGAAGGTGAGGAGCGT GAACAGCCTGATGGTGGCCAGCCTGACCTACAAGGACAAA GTGTACCTGAACGTGACCACCGACAACACCAGCCTGGATG ATTTTCACGTGAACGGAGGCGAGCTGATCCTGATCCATCAG AACCCCGGCGAGTTCTAA（SEQ ID NO:12） |

### 1.3. Construction of recombinant novel coronavirus high-efficiency immune DNA vaccine

As shown in Figure 5, the expression vector backbone pZDVac-IRES-OX40L obtained in the above step 1.1 was connected to the first target gene CRSNP obtained in the step 1.2 by T4 ligase, and the recombinant expression vector carrying the first target gene fragment CRSNP was obtained by colony PCR to screen positive clone: pZDVac-CTB-TT-PARDE-G6-RBD-(G4S)₂-S2-Foldon-CPPCP-Furin2A-ERISS-NP-IRES-OX4 0L (named pZDVac-CRSNPO recombinant plasmid, wherein the nucleotide sequence corresponding to fusion gene CTB-TT-PARDE-G6-RBD-(G4S)₂-S2-Foldon-CPPCP-Furin2A-ERISS-NP-IRES-OX40L is set forth in SEQ ID NO: 13, in addition to the nucleotide sequences shown in Table 1 above, it also contains restriction sites). The recombinant expression vector was the recombinant novel coronavirus high-efficiency immune DNA vaccine ZD-nCor19, and the specific construction steps included:

### 1.3.1. T4 ligation

The expression vector backbone pZDVac-IRES-OX40L (about 12kb) obtained by the gel recovery in the above step 1.1 was connected to the first target gene fragment CRSNP (about 3.6kb) obtained by the gel recovery in the step 1.2, and the ligation product was obtained overnight at 16 °C. The system was shown in Table 2.

**Table 2: T4 ligation system**

| Reagents Name | Dosage |
|---|---|
| Expression vector backbone pZDVac-IRES-OX40L | 4µL (20 ng) |
| First target gene fragment CRSNP | 8µL (600 ng) |
| ligase 10×NEB buffer | 2µL |
| T4 DNA ligase | 2µL (200 U) |
| ddH₂O | 4µl |
| Total volume | 20µL |

### 1.3.2. Screening of positive clones

100µL of stable competent cells were placed in an ice bath. After the competent cells were thawed, 10µL of the ligation product obtained in the above step 1.3.1 was added into the competent cell suspension, mixed gently, and placed in an ice bath for 30 minutes, then immediately transferred to a 42°C water bath for heat shock for 90s, and then quickly transferred to an ice bath for 2 min. 500 µL of sterile antibiotic-free LB medium was added to the transformed cells, mixed evenly, and then incubated with shaking at 150 rpm for 60 min at 37°C, and then centrifuged at 12,000 rpm for 1 min, and 500 µL of supernatant was discarded. 100 µL of transformed cells were taken and evenly spread on LB agar plate containing kanamycin (50 µg/mL), and cultured at 37°C for 48 hours in an inverted petri dish.

Single colonies were picked and screened positive clones by colony PCR. Upstream primer F and downstream primer R were designed for PCR amplification (primer sequences were synthesized by Beijing Biomed):
Upstream primer F: CCCAGGAGACCCGGTTCTAGAGACGGACATT (SEQ ID NO: 14);
Downstream primer R: AAGCGGCTTCGGCCAGTAACGTTAGGGGGGG (SEQ ID NO: 15);

The PCR system (25 µL) was: 12.5 µL of 2×PCR mix (purchased from Beijing Biomed); 11.5 µL of ddH₂O; 0.5 µL of F (10 µM) and 0.5 µL of R (10 µM). The PCR reaction conditions for PCR amplification of the ligation product transformed colonies were 95°C for 5 min, 1 cycle; 94°C for 30s, 55°C for 30s, and 72°C for 50s, 35 cycles; and 72°C for 7 min. PCR products were analyzed by 0.45% agarose DNA gel electrophoresis, and the results were shown in Figure 6, where in Lanes 1-5 were PCR results of different single colonies.

### 1.3.3. Plasmid extraction and restriction enzyme digestion identification of positive clones

Positive colonies screened by colony PCR were streaked on LB agar plate containing kanamycin (50 µg/mL) and cultured overnight at 37°C in an inverted petri dish. Then a single clone was picked and inoculated in 1.5mL LB medium containing kanamycin (50 µg/mL),cultured overnight at 37°C and 200rpm, then the obtained bacterial liquid was inoculated in 20mL LB medium containing kanamycin (50 µg/mL) at a ratio of 1:50, and cultured overnight at 37°C and 200rpm. In a sterile environment, 12 mL of the bacterial liquid was taken and prepared into glycerol bacteria based on the ratio of 1.2 mL bacterial liquid + 0.6 mL of 60% glycerol. The remaining bacterial liquid was centrifuged to extract plasmids, which were identified by single and double digestion with *AclI* and *Xbal* respectively. The single and double digestion systems were shown in Tables 3 and 4 below.

**Table 3: Single digestion system**

| Reagents Name | Dosage |
|---|---|
| Plasmid extracted from a positive clone | 7µL |
| restriction enzyme *AclI* or *XbaI* | 0.5µL |
| Cutsmart (10×) | 2µL |
| ddH₂O | 10.5µL |
| Total volume | 20µL |

**Table 4: Doubledigestion system**

| Reagents Name | Dosage |
|---|---|
| Plasmid extracted from a positive clone | 7µL |
| restriction enzyme *AclI* and *XbaI* | 0.5µL+0.5µL |
| Cutsmart (10×) | 2µL |
| ddH₂O | 10µL |
| Total volume | 20µL |

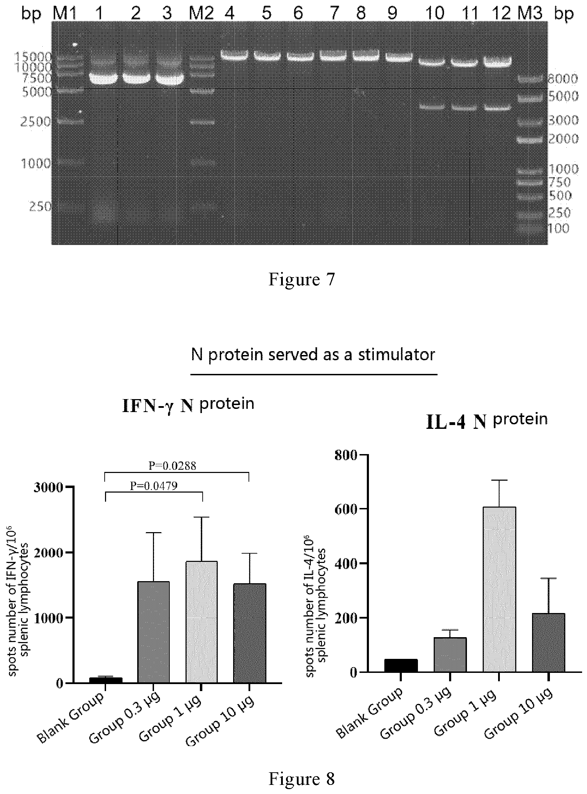

The samples were mixed according to the enzyme digestion system in Table 3 or 4 above, and centrifuged briefly to allow the sample and enzyme(s) to sink to the bottom of the tube. The centrifuge tubes were placed in a 37°C water bath and incubated for 2h. The bands of the expected size were identified by 0.45% agarose gel electrophoresis, and the identification results were shown in Figure 7, wherein Lanes 1-3 were the results of plasmids from the positive clones 1, 2, and 4 screened by colony PCR in Figure 6, respectively; Lanes 4-6 were the results of *AclI* single digestion of plasmids from the positive clones1, 2, and 4, respectively; Lanes7-9 were the results of *Xbal* single digestion of plasmids from the positive clones1, 2 and 4, respectively; Lanes 10-12 were the results of *AclI* and *XbaI* double digestion of plasmids from the positive clone 1, 2 and 4, respectively, including a band of about 12kb in size and a band of about 3.6kb in size, which proved that the recombinant plasmid pZDVac-CRSNPO, namely recombinant novel coronavirus high-efficiency immune DNA vaccine, was constructed.

In this example, a recombinant plasmid pZDVac-RBD-(G4S)₂-S2-NP (in which the empty vector pZDVac was used as the backbone vector, named as Control Plasmid 1) carrying the second target gene (RBD-(G4S)₂-S2-NP), and the recombinant plasmid pZDVac-Foldon-CPPCP-TT-PARDE-G6-RBD-(G4S)₂-S2-CTB-Furin2A-ERISS-NP-IRES-OX4 0L(named as Control plasmid 2) carrying the third target gene (Foldon-CPPCP-TT-PARDE-G6-RBD-(G4S)₂-S2-CTB-Furin2A-ERISS-NP) were constructed in the way similar as the way in step 1.3.

### Example 2: Immunogenicity induced by pZDVac-CRSNPO recombinant plasmid in mouse model

In this example, the pZDVac-CRSNPO recombinant plasmid constructed and obtained in the above Example 1 was used to verify its immunogenic effect induced in the mouse model, specifically including the following steps.

2.1. Twenty 6-8-week-old SPF female BALB/c mice (purchased from Beijing Weitong Lihua Laboratory Animal Technology Co., Ltd.) were randomly divided into 4 groups, with 5 mice in each group, and were immunized 3 times by intramuscular injection + electrical pulse stimulation, and the immunization interval was 7 days. The experimental groups and doses are shown in Table 5.

**Table 5: Grouping of pZDVac-CRSNPO recombinant plasmid in mouse model cellular immune response detection experiments**

| Group | Vaccine plasmid | Immunizing dose | Vaccination strategies | Number of animals |
|---|---|---|---|---|
| Bland Group | PBS | 100µL | intramuscul | 5 mice / group |
| Group 0.3µg | pZDVac-CRSNPO | 0.3µg/100µL | ar injection + electrical | |
| Group 1µg | pZDVac-CRSNPO | 1µg/100µL | pulse | |
| Group 10µg | pZDVac-CRSNPO | 10µg/100µL | stimulation | |

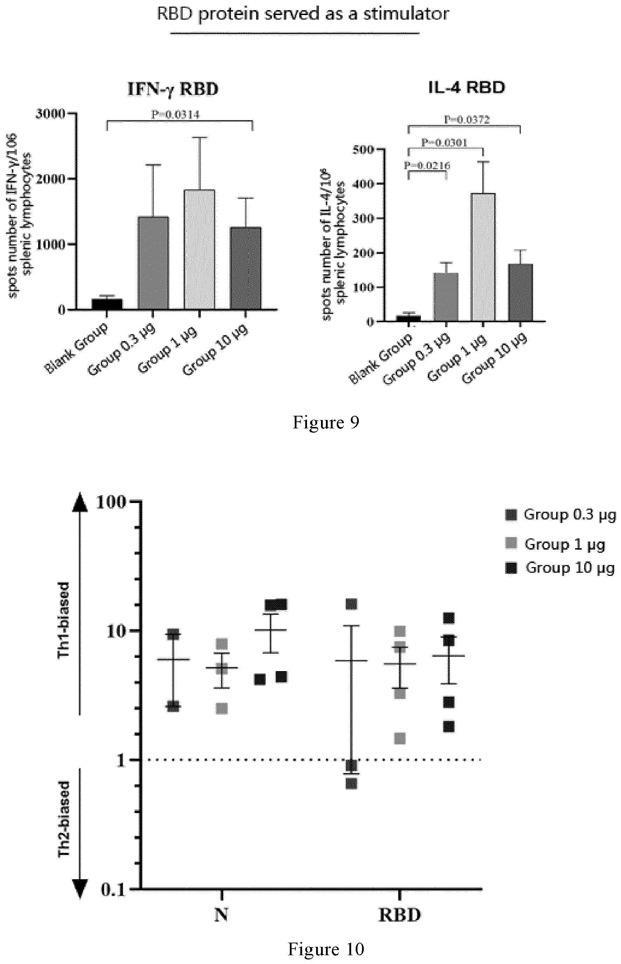

2.2. 2 weeks after the last immunization, the mice were sacrificed, and the spleen lymphocytes were isolated. After culturing for20 hours with the stimulation of the N protein and RBD protein of the novel coronavirus, immunology detection was performed according to the instructions of MabTech's Mouse IFN-γ and IL-4 ELISPOT plates. The statistical results of the secretion frequency detection of cytokines IFN-γ and IL-4 under different protein stimulators were shown in Figure 8 and Figure 9 respectively (where p<0.05 is statistically significant). The statistical results of antigen-specific T cell immune response bias were shown in Figure 10.

The results shown in Figure 8 to Figure 10 showed that different doses of pZDVac-CRSNPO recombinant plasmid could induce N antigen and RBD antigen-specific T cell immune responses in mice after immunization, and secrete not only IFN-γ representing Th1-type response, but also IL-4 representing Th2-type response, wherein the secretion frequency of Th1-type cytokine IFN-γ was significantly higher than that of Th2-type cytokine IL-4. The results indicated that the T cell immune response induced by the pZDVac-CRSNPO recombinant plasmid was biased towards Th1, and compared with Group 0.3 µgand Group 10 µg, 1 µg was the optimal immune dose.

According to the same method as shown in the above steps 2.1 and 2.2, the immunogenicity induced by immunization of 6-8-week-old SPF female BALB/c mice with Control plasmid 1 and Control plasmid 2 was detected respectively. The results showed that the immunogenic effect induced by Control plasmid 1 and Control plasmid 2 was not as good as that induced by the pZDVac-CRSNPO recombinant plasmid.

### Example 3: Immunogenicity detection of pZDVac-CRSNPO recombinant plasmid in humans

In this example, the pZDVac-CRSNPO recombinant plasmid constructed and obtained in Example 1 was used to immunize human subjects, and the immunogenicity effect induced in the human body was verified, including the following steps.

3.1. 15 selected volunteers (internal personnel of the applicant unit, who have signed to volunteer to participate in the trial) were randomly divided into 3 groups with 5 people in each group. The immunization interval was 28 days, and the immunization was carried out three times. Before the first immunization, 14 days after the second immunization and 21 days after the third immunization, PBMCs from the volunteers were taken for ELISPOT detection. The first two immunizations were intramuscular injection + electrical pulse stimulation, and the third immunization was intradermal injection + electrical pulse stimulation. The experimental groups and doses are shown in Table 6 below.

**Table 6: Grouping of pZDVac-CRSNPO recombinant plasmid in the detection of cellular immune response in human subjects**

| Group | Vaccine plasmid | Immunizing dose | Number of volunteers |
|---|---|---|---|
| Low dose group | pZDVac-CRSNPO | 100µg | 5persons / group |
| Medium dose group | | 300µg | |
| High dose group | | 500µg | |

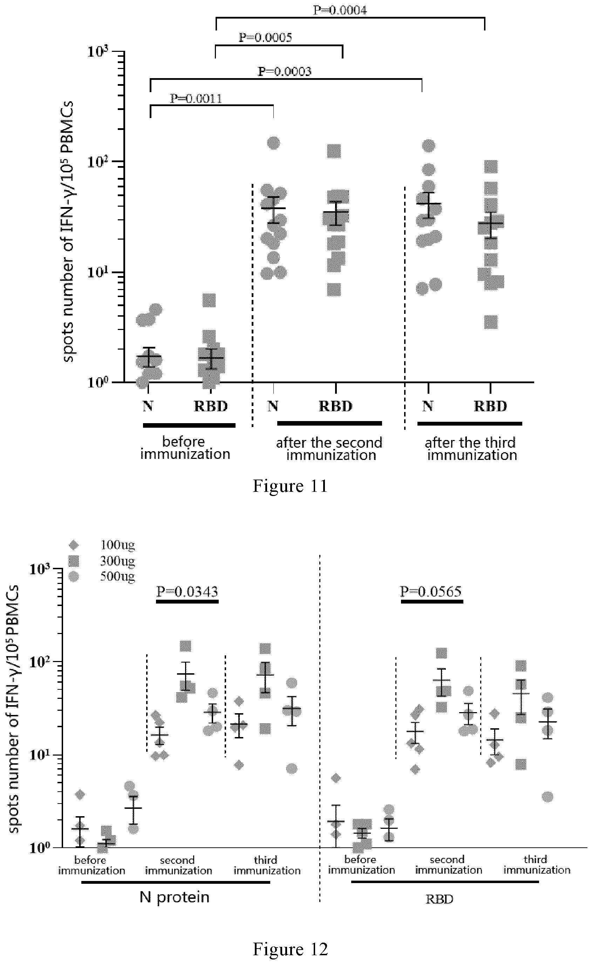

3.2. The fresh PBMCs obtained from the volunteers were taken, and stimulated and cultivated with N protein and RBD protein of the novel coronavirus for 20 hours, and immunological detection was performed according to the instructions of Human IFN-γ ELISPOT plates of MabTech Company. The statistical results of the secretion frequency detection of cytokine IFN-γ under different protein stimulators after three immunizations were shown in Figure 11 (whereinp<0.05 means the statistical difference is significant; p<0.01 means the statistical difference is very significant). The statistical results of the three immunizations responses with different dose were shown in Figure 12 (wherein p<0.05 means that the statistical difference is significant; p<0.01 means that the statistical difference is very significant).

The results in Figure 11 showed that compared with that before immunization, strong cellular immune responses could be induced after secondary and tertiary immunization, and the secretion frequency of cytokine IFN-γ in PBMCs had very significant increase when N protein and RBD protein were used as stimulators, respectively; and the secretion frequency of the cytokine IFN-γ after the third immunity was higher than that after the second immunity when the N protein was used as a stimulator, and the secretion frequency of the cytokine IFN-γ after the second immunity was higher than that after the third immunity when the RBD protein was used as a stimulator. The results in Figure 12 showed that the number of spots in different dose groups increased significantly after immunization compared with that before immunization, indicating that specific cellular immunity could be effectively induced at each dose; while there were significant difference(p=0.0343, which is the result of variance analysis among the three dose groups of low, medium and high doses) in the number of spots among the dose groups after the second immunization when N protein and RBD protein was used as a stimulator. It could be seen that compared with the high-dose group and the low-dose group, the immune effect of the medium-dose group was better, and the number of spots did not increase much after the third immunization. Therefore, when the pZDVac-CRSNPO recombinant plasmid is used as a new coronavirus DNA vaccine, 2 immunizations at medium-dose (for example, 28 days between 2 immunizations) can be used.

In addition, the 15 volunteers screened in this example underwent regular follow-up and physical examination (at least 3 months) after the vaccine injection, and all volunteers did not show any discomfort, which preliminarily shows that the pZDVac-CRSNPO recombinant plasmid provided by the present invention has a good safety profile when used as a vaccine.

### Example 4: Immune routes and electro-introduction conditions of pZDVac-CRSNPO recombinant plasmid

In this example, the pZDVac-CRSNPO recombinant plasmid constructed and obtained in Example 1 was used to immunize mice through different immunization routes (intramuscular injection or intradermal injection) and under different electro-introduction conditions to evaluate the intensity of the immune response induced through different immunization routes and under the different electro-introduction conditions, specifically including the following steps.

4.1. According to the groups shown in Table 7 below, 40 healthy 6-8-week-old female Balb/c mice (purchased from Viton Lever) were randomly divided into 5 groups, 8 mice in each group, respectively. The mice in each group were immunized by single-point administration to the left hind limb of mice by means of intramuscular injection or intradermal injection &electro-introduction (1Hz×6 times, 0.5 cm of electrode needle spacing), respectively. A blank control group was set without treatment, and mice in other groups were given 1 µg/mice of pSFVK1-CRSNPO recombinant plasmid. The dosing day was counted as 0 d, and 5 groups of mice were immunized twice on 0 d and 21 d respectively.

**Table 7: Grouping of mice under different immunization routes and electro-introduction conditions of pZDVac-CRSNPO recombinant plasmid**

| Group | voltage | pulse width |
|---|---|---|
| Blank control group | -- | -- |
| Intramuscular injection group | 60V | 50ms |
| Intradermal injection group a | 60V | 50ms |
| Intradermal injection group b | 36V | 50ms |
| Intradermal injection group c | 36V | 10ms |

4.2. The serum was taken 2 weeks after the last immunization for antibody detection. Specifically, ACRO Biosystems antibody detection kit for serum antibody (RBD IgG) detection was used. The mouse serum was diluted at 1:100, and positive judgment was the absorbance value at OD450nm>0.1.2 weeks after the last immunization, mice in each group were sacrificed, and ELISPOT detection (IFN-γ) was performed according to the instructions of MabTech's IFN-γ ELISPOT plate. The expression method of spot count in ELISPOT experiment was: spot-forming units(SFU)/10⁶ splenocytes; the criteria for judging the experimental wells as positive was: the number of spots in the experimental wells > the average spot value of the negative control wells + 2SD; the calculation method for the number of positive spots in each mouse was as follows: 5 duplicate wells were made for the spleen cells of one mouse, and if at least 3 wells were positive, then the data of this mouse would be valid, and the number of spots in this mouse = the average number of spots in experimental wells - the number of spots in the negative control wells; the calculation method of the number of spots in each group of mice was as follows: the data of each effective mouse in the group was averaged and expressed as the mean ± standard deviation. Statistical analysis of the data was performed with GraphPad Prism 8 software, wherein the differences between two groups were analyzed with Student's t test, and the differences among multiple groups were compared with one-way analysis of variance. p<0.05 means that the difference is statistically significant, and p<0.01 means that the difference is very significant.

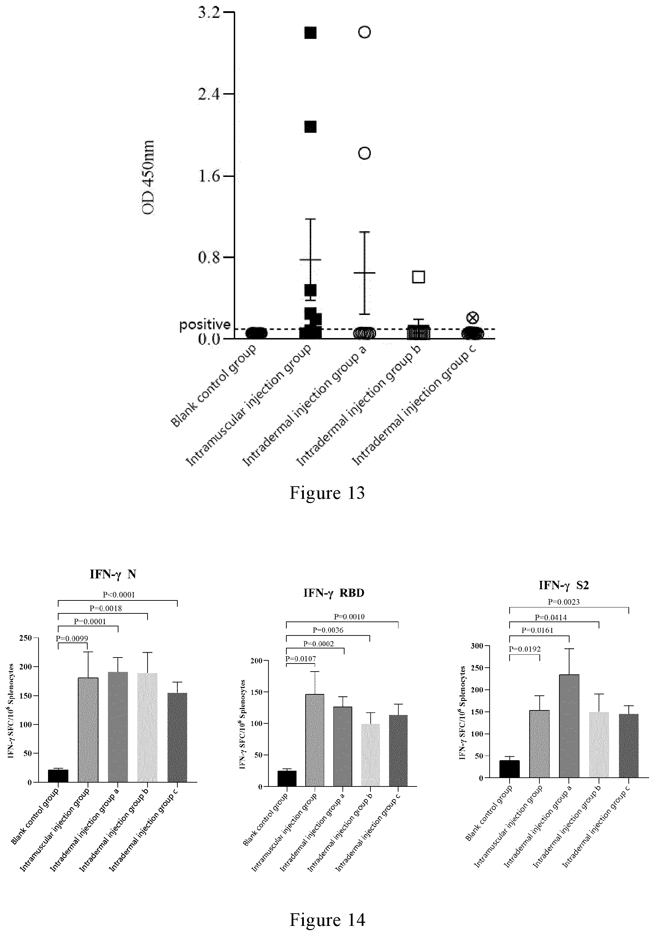

The results of antibody detection were shown in Figure 13. It could be seen that the pSFVK1-CRSNPO recombinant plasmid can induce antibody responses after immunizing mice through different routes (intramuscular injection and intradermal injection) or under different electro-introduction conditions. Compared with the blank control group, the antibody level induced by the intramuscular injection group (60V, 50ms, 1Hz×6 times, 0.5cm of electrode needle spacing, and 120V/cm of field strength) was statistically different; the antibody level induced by the intradermal injection group a (60V, 50ms, 1Hz×6 times, 0.5cm of electrode needle spacing, 120V/cm of field strength) was also statistically significantly different from that of the blank control group; while the antibody levels induced by the intradermal injection group b (36V, 50ms), and c (36V, 10ms) had no statistically significant difference compared with the blank control group. These results showed that under the conditions of intramuscular injection and subcutaneous injection, compared with the electro-introduction conditions with voltage of 36V, and pulse width of 50ms,it had a relatively better immune effect when the electro-introduction conditions were 60V of voltage, 50ms of pulse width. In addition, under the same electro-introduction conditions, the antibody level induced by intradermal injection group a was not statistically different from that of intramuscular injection group, but in general, immunization with pSFVK1-CRSNPO recombinant plasmid through intramuscular injection route had relatively better humoral immunity effect.

The ELISPOT test results were shown in Figure 14. It could be seen that the pSFVK1-CRSNPO recombinant plasmid could induce N antigen, RBD antigen and S2 antigen-specific T cell immune responses after immunizing mice with different routes (intramuscular injection and intradermal injection) and under different electro-introduction conditions. The experimental groups (intramuscular injection group, subcutaneous injection group a, b, c) were all statistically significantly different from the blank control group. Among them, the N antigen, RBD antigen and S2 antigen-specific T cells immune responses induced by immunization by intradermal injection under various electro-introduction conditions were different from those by immunization by intramuscular injection.

To sum up, the results showed that the pSFVK1-CRSNPO recombinant plasmid vaccine provided by the present invention could induce strong specific T cell immune responses in mice stimulated through different immunization routes and under different electro-introduction conditions, and there was no significant difference in the induced cellular immune response between the intramuscular injection group and the intradermal injection group. The pSFVK1-CRSNPO recombinant plasmid vaccine provided had a relatively better induction effect in terms of inducing humoral immune response under the conditions of voltage 60V and pulse width 50ms, and in general, vaccine administered intramuscularly produced relatively better humoral immune response. Therefore, the pSFVK1-CRSNPO recombinant plasmid provided by the present invention can induce a good cellular immune response and humoral immune response when used as a vaccine, and can exert a good protective effect of vaccination, which is consistent with the results of Examples 2 and 3 above.

### Example 5: Immune challenge test

In this example, according to the vaccine immunization method in the above-mentioned Example 4 (intramuscular injection or subcutaneous injection &electro-introduction (60V, 50ms, 1Hz×6 times, 0.5cm of electrode needle spacing, 120V/cm of field strength)), pSFVK1-CRSNPO recombinant plasmid was used to immunize hACE2 transgenic mice or rhesus monkeys, and a negative control group was set up and given normal saline. The dosing day was counted as 0 d, and the animals in each group were immunized twice on 0 d and 21 d respectively, and the animals in each group were challenged (novel coronavirus liquid) 2 weeks after the last immunization. The reduction of pulmonary viral load and the pulmonary pathological conditions of the animals in each group were investigated, and the reduction of pulmonary viral load (≥2 logs) and the improvement of pulmonary pathology were the basic requirements for effectiveness evaluation. The results showed that the animals in the negative control group showed typical clinical symptoms of novel coronavirus infection (such as fever, lethargy, weight loss, and even death) after the challenge, while at least 4/5 of the animals in the immunized group were protected, without showing any of typical clinical symptoms of novel coronavirus infection, and the pulmonary viral load reduced (≥2 logs) and the pulmonary pathology was significantly improved. These results show that the recombinant novel coronavirus high-efficiency immune DNA vaccine ZD-nCor19 provided by the present invention has effectiveness and safety.

To sum up, the results of the above examples showed that the recombinant novel coronavirus high-efficiency immune DNA vaccine (that is, the pZDVac-CRSNPO recombinant plasmid) constructed and obtained by the present invention can effectively induce high-efficiency immune responses in animal-level immunological function tests, including natural immune responses and antigens-specific cellular immune response, which proves that it is a new type of coronavirus DNA vaccine with great development potential. After using the vaccine to immunize human subjects, it was found that it could induce high levels of natural immune responses and antigen-specific cellular immune responses in all three dose groups (100 µg, 300 µg, 500 µg), and the highest level of immune response was induced in the 300 µg dose group. All human subjects who participated in the test did not have any obvious adverse reactions such as fever, allergy, headache, general weakness, etc. during regular follow-up and physical examination. By comparing the strength of the immune response induced through different immunization routes and under different electro-introduction conditions, it shows that the recombinant novel coronavirus high-efficiency immune DNA vaccine provided by the present invention can be inoculated through intramuscular injection and subcutaneous injection, and both can induce good specific T cell immune responses and humoral immune responses. The results of the immune challenge test also shows that the recombinant novel coronavirus high-efficiency immune DNA vaccine of the present invention has good safety and immune protection effects. The above results all preliminarily confirm that the vaccine constructed by the present invention is a kind of vaccine that can induce high-level immune response and at the same time is very mild and safe.

The examples described herein are for illustration (as an example) only, and various modifications or changes made by the skilled person should also be included within the essential scope of the patent application.

### Industrial applicability

The present invention provides a fusion gene and a recombinant novel coronavirus high-efficiency immune DNA vaccine based on the fusion gene and a construction method and use thereof, wherein the recombinant novel coronavirus high-efficiency immune DNA vaccine has the dual effects of preventing and treating novel coronavirus infection, suitable for industrial applications.

## Claims

1. A fusion gene, comprising at least two of the following (1) to (4):
(1) a gene expressing RBD segment of novel coronavirus COVID-19;
(2) a gene expressing S2 subunit or partial fragment thereof of the novel coronavirus COVID-19;
(3) a gene expressing N protein or partial fragment thereof of the novel coronavirus COVID-19;
(4) a gene expressing an amino acid fragment selected from the group consisting of CTB, TT, PADRE, Foldon, CPPCP, Furin2A, ERISS, IRES, and OX40L, or combination thereof.

2. The fusion gene of claim 1, comprising at least three of the following (1) to (4):
(1) a gene expressing the RBD segment of the novel coronavirus COVID-19;
(2) a gene expressing residues 301-538 in the S2 subunit of the novel coronavirus COVID-19;
(3) a gene expressing residues 138-369 in the N protein of the novel coronavirus COVID-19;
(4) genes expressing the following amino acid fragments: CTB, TT, PADRE, Foldon, CPPCP, Furin2A, ERISS, IRES and OX40L.

3. The fusion gene of claim 2, wherein the gene expressing the RBD segment and the gene expressing the residues 301-538 in the S2 subunit are linked to form a fusion fragment;
preferably, the nucleotide sequence of the fusion fragment comprises the sequence set forth in SEQ ID NO: 6; the nucleotide sequence of the gene expressing the residues 138-369 in the N protein comprises the sequence set forth in SEQ ID NO: 10.

4. The fusion gene of any one of claims 1-3, wherein:
(1) the nucleotide sequence of the gene expressing the CTB amino acid fragment is set forth in SEQ ID NO: 2;
(2) the nucleotide sequence of the gene expressing the TT amino acid fragment is set forth in SEQ ID NO: 3;
(3) the nucleotide sequence of the gene expressing the PADRE amino acid fragment is set forth in SEQ ID NO: 4;
(4) the gene expressing the Foldon amino acid fragment and the gene expressing the CPPCP amino acid fragment are linked to form a synthetic fragment, and the nucleotide sequence of the synthetic fragment is set forth in SEQ ID NO:7;
(5) the nucleotide sequence of the gene expressing the Furin2A amino acid fragment is set forth in SEQ ID NO: 8;
(6) the nucleotide sequence of the gene expressing the ERISS amino acid fragment is set forth in SEQ ID NO: 9;
(7) the nucleotide sequence of the gene expressing the IRES amino acid fragment is set forth in SEQ ID NO: 11; and/or
(8) the nucleotide sequence of the gene expressing the OX40L amino acid fragment is set forth in SEQ ID NO: 12.

5. The fusion gene of any one of claims 2-4, wherein:
the gene expressing the RBD segment and the gene expressing the residues 301-538 in the S2 subunit are linked to form a fusion fragment, the upstream of the fusion fragment is sequentially linked with the genes expressing the amino acid fragments of CTB, TT and PADRE, and the downstream of the fusion fragment is sequentially linked with the genes expressing the amino acid fragments of Foldon, CPPCP and Furin2A; and/or
the gene expressing the ERISS amino acid fragment is linked upstream of the gene expressing the residues 138-369 in the N protein, and the genes expressing the IRES and OX40L amino acid fragments are sequentially linked downstream of the gene expressing the residues 138-369 in the N protein;
preferably, the gene expressing the RBD segment and the gene expressing the residues 301-538 in the S2 subunit are linked by a gene expressing a (G4S)₂ linker, the upstream of the fusion fragment and the gene expressing the PADRE amino acid fragment are linked by the a gene expressing a linker G6, and the nucleotide sequence of the gene expressing the linker G6 is set forth in SEQ ID NO:5;
further preferably, the nucleotide sequence of the fusion gene is set forth in SEQ ID NO: 13.

6. A fusion protein obtained from the expression of the fusion gene of any one of claims 1-5.

7. A recombinant novel coronavirus high-efficiency immune DNA vaccine, named ZD-nCor19, comprising the fusion gene of any one of claims 1-5 and a vector.

8. The recombinant novel coronavirus high-efficiency immune DNA vaccine of claim 7, wherein the vector is pZDVac vector.

9. A method for constructing the recombinant novel coronavirus high-efficiency immune DNA vaccine of claim 7 or 8, comprising the following steps of
1) synthesizing the fusion gene of any one of claims 1-5;
2) inserting the fusion gene into a pZDVac vector to obtain the recombinant novel coronavirus high-efficiency immune DNA vaccine.

10. The recombinant novel coronavirus high-efficiency immune DNA vaccine of claim 7 or 8 for use in the preparation of a medicine for preventing and/or treating novel coronavirus infection.
